(19) Europäisches Patentamt
European Patent Office
Office européen des brevets

(11) Veröffentlichungsnummer: **0 275 910 B1**

(12) # EUROPÄISCHE PATENTSCHRIFT

(45) Veröffentlichungstag der Patentschrift: **16.10.91**

(51) Int. Cl.5: **C07C 215/76**, C07C 211/55, C07C 217/76, C07D 279/20, C08K 5/17, C08K 5/46

(21) Anmeldenummer: **88100372.7**

(22) Anmeldetag: **13.01.88**

(54) **Neue Stabilisatoren und deren Verwendung zur Herstellung stabilisierter Polyamide und Kautschukmaterialien.**

(30) Priorität: **22.01.87 DE 3701738**

(43) Veröffentlichungstag der Anmeldung:
**27.07.88 Patentblatt 88/30**

(45) Bekanntmachung des Hinweises auf die Patenterteilung:
**16.10.91 Patentblatt 91/42**

(84) Benannte Vertragsstaaten:
**DE FR GB IT**

(56) Entgegenhaltungen:
**EP-A- 0 070 436**
**US-A- 3 867 334**

**CHEMICAL ABSTRACTS, Band 77, Nr. 24, 11. Dezember 1972, Seite 71, Nr. 153541s, Columbus, Ohio, US; & JP-A-72 19 088**

(73) Patentinhaber: **BAYER AG**

**W-5090 Leverkusen 1 Bayerwerk(DE)**

(72) Erfinder: **Buysch, Hans-Josef, Dr.**
**Brandenburger Strasse 28**
**W-4150 Krefeld(DE)**
Erfinder: **Szentivanyi, Zsolt, Dr.**
**Carl-Rumpff-Strasse 35**
**W-5090 Leverkusen(DE)**
Erfinder: **Brassat, Bert, Dr.**
**Bodelschwinghstrasse 30**
**W-4150 Krefeld(DE)**
Erfinder: **Oppenheimer-Stix, Christiane, Dr.**
**Dörperhofstrasse 15**
**W-4150 Krefeld(DE)**

**EP 0 275 910 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Stabilisatoren bzw. Stabilisatorgemische, die nach einem Verfahren der Umsetzung von Isopropenylphenolen oder Bisphenolen vom Typ Bisphenol A mit Diphenylamin oder Phenothiazin oder Derivaten dieser Verbindungen in Gegenwart von sauren Katalysatoren enthalten werden und gegebenenfalls mit Oxiranen, Aldehyden und anderen Verbindungen weiter abgewandelt werden können. Ferner wird deren Verwendung zur Herstellung von mit diesen Substanzen stabilisierten Polyamiden und Kautschukmaterialien beansprucht.

Diphenylaminderivate mit angegliederten Phenolkernen sind grundsätzlich bekannt. In der US-PS 3 673 091 sind Diphenylamine der Struktur I

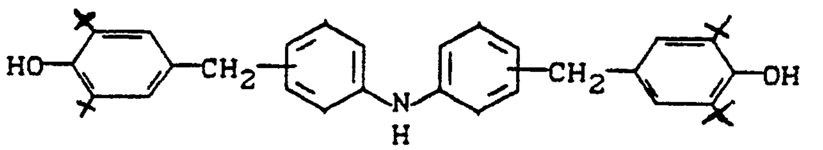

(I)

beschrieben, hergestellt aus Diphenylamin und 4-Hydroxy-3,5-di-tert.-butyl-benzylalkohol, die als Antioxidantien für Schmieröle verwendet werden. Ihre antioxidative Wirkung in anderen Substraten wie Polyamiden ist jedoch nur mäßig oder ganz unzureichend.

Es wurde nun überraschend gefunden, daß verfahrensgemäße Umsetzungsprodukte aus Isopropenylphenolen oder Bisphenolen mit Diphenylamin, Phenothiazin oder Derivaten dieser Verbindungen ausgezeichnete Antioxidantien für Polyamide und Kautschukmaterialien darstellen.

Besonders überraschend ist, daß Produkte dieser Art, auch wenn sie keine polymerisierbaren Funktionen enthalten, sich im Kautschuk wie gebundene Stabilisatoren verhalten und daher auch unter extraktiven Bedingungen ihre Wirkung länger behalten als übliche Antioxidantien.

Gebundene Antioxidantien sind über Haftgruppen an die Polymermatrix chemisch fixiert. Solche Haftgruppen stellen z.B. die Nitroso-, Sulfhydryl-, Vinyl-, Allyl-und Acrylgruppe dar. Alterungsschutzmittel dieser Art sind beschrieben in DE-A 2 735 178, DE-A 2 509 654, DE-A 3 022 952, DE-A 2 025 336, DE-A 3 113 351, US-PS 3 867 334.

Die erfindungsgemäßen Alterungsschutzmittel behalten einerseits selbst unter extraktiven Alterungsbedingungen ihre hervorragende Schutzwirkung, wenn sie keine Haftgruppen aufweisen. Andererseits ist es jedoch auch möglich, sie mit solchen Haftgruppen auszurüsten, wie weiter unten gezeigt wird.

Geeignete Ausgangsprodukte zur Herstellung der neuen Stabilisatoren sind Diphenylamine der allgemeinen Struktur (II) und Phenothiazine der allgemeinen Struktur (III)

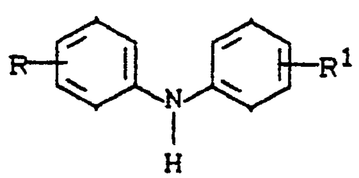

(II)

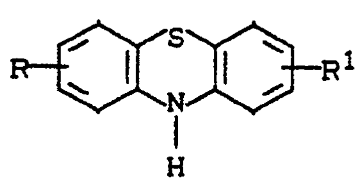

(III)

worin

R und $R^1$ gleich oder verschieden sind und in ortho-, meta- und/oder para-Position zum N-Atom stehen und $C_1$ bis $C_8$-Alkyl-, $C_7$ bis $C_{10}$-Aralkyl-, OH-oder Cl-Reste, bevorzugt $CH_3$-, $C_2H_5$-, tert.-Butyl-, Isooctyl-, Styryl-oder OH-Reste, besonders bevorzugt aber Wasserstoff sind, ferner für Reste der Strukturen (IIa)

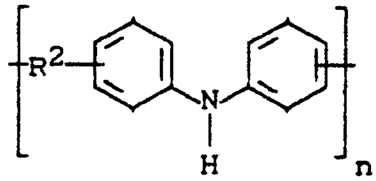

(IIa)

oder der Struktur (IIIa)

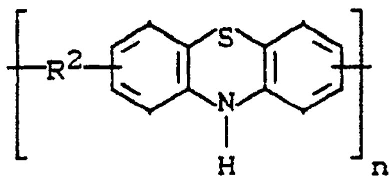

(IIIa) stehen,

worin
$R^2$ für einen zweiwertigen Rest

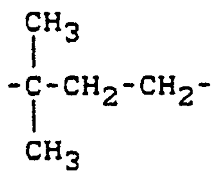

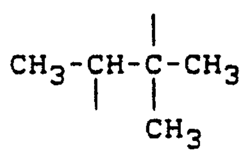

,

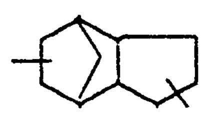

,

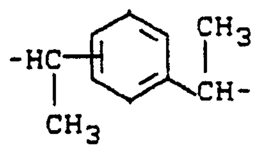

,

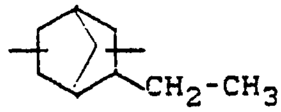

,

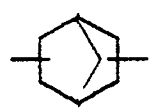

,

bevorzugt für

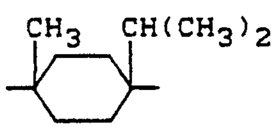

,

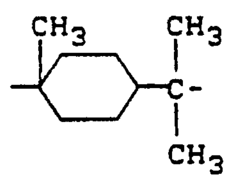

,

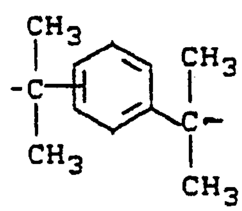

und

$-CH_2-$, steht,
wobei die Reste (IIa) und (IIIa) über $R^2$ mit den Resten (II) bzw. (III) verbunden sind und n eine ganze Zahl von 1 bis 5, bevorzugt 1 bis 3 bedeutet.

Ausgangsprodukte mit den Strukturen (IIa) und (IIIa) und ihre Herstellung sind in der EP 70436 beschrieben. Bevorzugt werden jedoch Verbindungen der Struktur (II) und (III) eingesetzt, besonders bevorzugt Diphenylamin und Phenothiazin.

Als phenolische Ausgangsverbindungen dienen Isopropenylphenole der Struktur (IV)

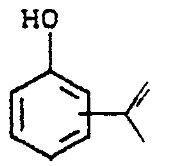

(IV)

worin die Isopropenylgruppe o-, m-, bevorzugt aber p-ständig zur OH-Gruppe sein kann, oder Bisphenole der Struktur (V)

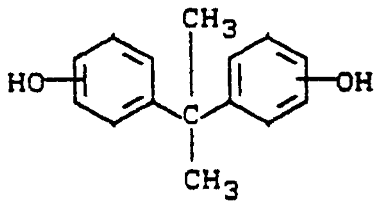

(V)

worin die OH-Gruppen in o-, m-, bevorzugt aber in p-Position zur Isopropylidengruppe stehen. Man kann auch das bei der Bisphenol-A-Herstellung anfallende Harz verwenden, das nach Kristallisation und Abtrennung von Bisphenol A als Rückstand der Mutterlauge gewonnen wird und noch relativ große Mengen an Bisphenolen der Struktur V enthält.

Die Umsetzung von Diphenylaminen der Struktur (II) oder Phenothiazinen der Struktur (III) mit Isopropenylphenolen (IV) oder Bisphenolen (V) erfolgt im erfindungsgemäßen Verfahren in Gegenwart eines sauren Katalysators in der Schmelze oder in (unter den Reaktionsbedingungen inerten) Lösungsmitteln bei Temperaturen zwischen 100 bis 300°, bevorzugt 130 bis 260°, besonders bevorzugt 150 bis 250°.

Geeignete Lösungsmittel sind aliphatische oder aromatische Kohlenwasserstoffe, aromatische Halogenverbindungen oder Ether. Genannt seien beispielsweise Isooctan, Cyclohexan, Decan, Toluol, Xylol, Mesitylen, Chlorbenzol, Dichlorbenzol, Diphenylether. Zweckmäßig verwendet man solche, die unter den Reaktionsbedingungen nicht flüchtig sind, so daß die Reaktion nicht in Druckgefäßen durchgeführt werden muß.

Die Reaktionskomponenten Amin und Phenol werden im Molverhältnis von 0,1:1 bis 2:1, bevorzugt von 0,3:1 bis 1,5:1 und besonders bevorzugt von 0,4:1 bis 1:1 eingesetzt.

Geeignete Katalysatoren sind solche, die einen in Wasser gemessenen $pK_{Säure}$-Wert kleiner als 3 aufweisen. Genannt seien z.B. Chlorwasserstoff, Phosphorsäure, Phosphorige Säure, Schwefelsäure, Methansulfonsäure, p-Toluolsulfonsäure, Methanphosphonsäure, Benzolphosphonsäure, Aluminiumchlorid, Borfluorid und seine Addukte wie Borfluorid-Etherat, Bortrichlorid. Weiterhin sind saure Aluminiumsilikate wie Zeolithe und säureaktivierte Schichtsilikate des Bentonit- und Montmorillonit-Typs geeignet.

Geht man von Verbindungen der Struktur (V) aus, so entsteht während der Umsetzung Phenol, das zweckmäßig während der Reaktion oder nach deren Beendigung abdestilliert wird.

Man erhält die Umsetzungsprodukte nach Abdestillieren des gegebenenfalls verwendeten Lösungsmittels und des gegebenenfalls gebildeten Phenols als kristalline oder harzige, häufig spröde braun gefärbte Rückstände. Naturgemäß bestehen letztere meist aus mehreren chemischen Individuen, die beispielsweise bei Umsetzung von Diphenylamin bzw. Phenothiazin mit Isopropenylphenol zum Teil den Strukturen (VI) bzw. (VII)

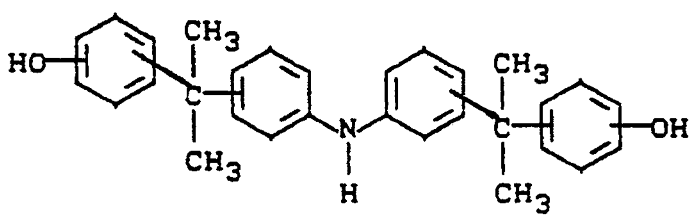

(VI)

beziehungsweise

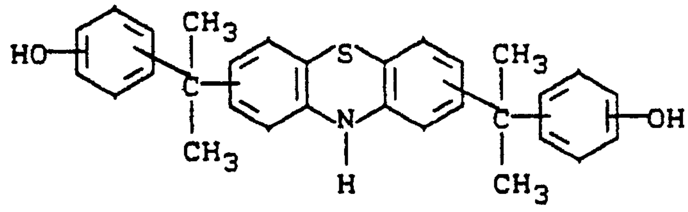

(VII)

entsprechen.

Die primären Umsetzungsprodukte, beispielhaft dargestellt durch (VI) und (VII), können weiter modifiziert werden durch Reaktion mit Verbindungen der Struktur (VIII)

$X[R^3\text{-}Y]$ (VIII)

worin

X und Y gleich oder verschieden sein können und H, OH, Halogen (besonders Cl oder Br), eine Ethylen-, Propylen-oder eine Oxirangruppe bedeuten, wobei auch einer der beiden Substituenten oder der Ausdruck in der Klammer H sein kann mit der Maßgabe, daß X im letzteren Fall von OH oder Halogen verschieden ist; ferner kann X für -CH=O stehen, wenn der Rest in der Klammer aliphatisch ist und 1 bis 4 Kohlenstoffatome aufweist,

$R^3$ ein ein- oder zweibindiger aromatischer Rest oder ein ein- oder zweibindiger aliphatischer Rest mit 1 bis 12 C-Atomen, der eine oder mehrere OH, Ether-, Thioether, Carbonyl, Estergruppen und Doppelbindungen sowie aromatische Kerne mit 6 bis 12 C-Atomen enthalten kann,

wobei diese Reaktion sowohl an den OH-Gruppen wie an den aromatischen Kernen der primären Umsetzungsprodukte erfolgen kann.

Bevorzugt stehen für X und Y, H, OH, Halogen, Ethylen, Propylen, Oxiran

X außerdem für -CH=O, wenn der Rest in der Klammer H oder $CH_3$ ist,

$R^3$ für einen 1- oder 2-bindigen aromatischen Rest mit 6 C-Atomen oder einen 1- oder 2-bindigen aliphatischen Rest mit 1 bis 9 C-atomen, der eine oder zwei Ether-, Estergruppen und/oder Doppelbindungen sowie aromatische Kerne mit 6 C-Atomen enthalten kann.

Besonders bevorzugt stehen X und Y für H, Halogen, Oxiran, X außerdem für -CH=O, wenn der Klammerausdruck Wasserstoff darstellt

$R^3$ für einen 1- oder 2-bindigen aliphatischen Rest mit 1 bis 6 C-Atomen.

Geeignete Ausgangsprodukte der Struktur (III) sind z.B. Aldehyde wie Formaldehyd, Acetaldehyd, Isobutyraldehyd, Alkylhalogenide wie Allylchlorid, Methallylbromid, Methylenchlorid, 1,4-Dichlorbuten-2, Benzylchlorid, Xylylendichlorid, Dichlorethan, Dibromhexan. Alkohole wie Isopropanol, tert.-Butanol, Amylalkohol, α-Hydroxyethylbenzol, α-Hydroxycumol, α,α'-Dihydroxydiisopropylbenzol, Olefine wie Propen, Buten, Isobuten, Isopren, 2,5-Dimethyl-hexadien-1,5, Divinylbenzol, Diisopropenylbenzol, Acrylsäureethylester, Methacrylsäuremethylester, Glykoldimethacrylat, Diglykoldiacrylat, Methylvinylketon, Carbonsäurechloride wie Acetylchlorid, Acrylsäurechlorid, Methacrylsäurechlorid, Fumarsäuredichlorid, Adipinsäuredichlorid, Terephthalsäuredichlorid, 4-Thia-heptandisäuredichlorid, 3-Oxa-pentandisäuredichlorid, 4,5-Dithia-octandisäuredichlorid, Oxirane wie Ethylenoxid, Propylenoxid, Glycidol, Epichlorhydrin, Diglycid, Bisphenolbisglycidylether, Vinylethylenoxid, N,N-Bisglycidylanilin und Hexahydrophthalsäurebisglycidylester.

Bevorzugt sind Formaldehyd, Allylchlorid, 1,4-Dichlorbuten-2, Benzylchlorid, tert. Butanol, α-Hydroxycumol, α,α'-Dihydroxyisopropylbenzol, Isobuten, Diisopropenylbenzol, (Meth)Acrylsäurechlorid, ferner besonders auch Oxirane wie Ethylenoxid, Propylenoxid und Epichlorhydrin.

Die Reaktion mit den Verbindungen der Struktur (VIII) wird nach üblichen Methoden durchgeführt. Alkylierungen mit Alkoholen oder Olefinen in Gegenwart von starken Säuren bei erhöhter Temperatur, Alkylierungen oder Acylierungen mit Halogenverbindungen durch bloßes Erhitzen oder aber in Gegenwart von äquimolaren Mengen an Basen, um aus dem phenolischen OH ein Phenolat mit erhöhter Reaktivität zu bilden und die freiwerdende Säure zu binden (vgl. Houben-Weyl, Methoden der organischen Chemie 6/1c, S. 925 ff und VIII, S. 543 ff).

Bei Verwendung von aktivierten Olefinen wie Acrylestern genügt für die Alkylierung der phenolischen OH-Gruppe nach dem Typ einer Michael-Addition eine katalytische Menge Base. Das gleiche gilt auch für die basisch katalysierte Addition von Oxiranen an die phenolische OH-Gruppen (Houben-Weyl, Methoden

der Organischen Chemie, Band VI/3, S. 49 ff, 79 ff und 6/1c S. 999 und 1081; 6/3, S. 71 ff und Rodds Chemistry of Carbon Compounds, Vol III E, 158 ff, Elsevier Publ. Co. Amsterdam 1974) und Organic Reactions Vol 10, S. 179 ff, John Wiley and Sons, 1959).

Ausgehend von den Strukturen (VI) und (VII) sei beispielhaft erläutert, zu welchen Strukturen man auf diese Weise gelangt:
Mit einen 1-bindigen Rest $R^3$ erhält man z.B.

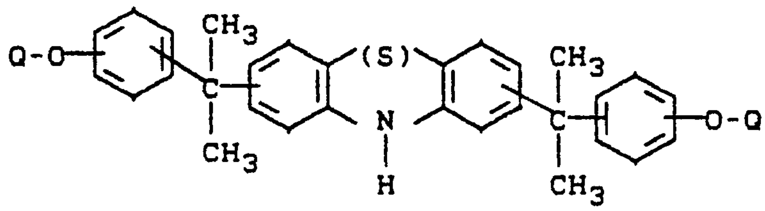

worin Q sein kann

$CH_3-$, $HO-CH_2-CH_2-$, $HO-CH-CH_2-$ ($CH_3$), 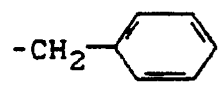 ,

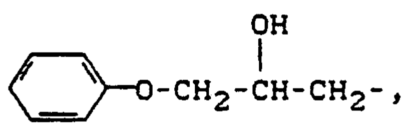, $CH_2=CH-CH_2-$, $CH_2=C-CH_2-$ ($CH_3$),

$CH_3CO-$, $CH_2=CH-CO-$, $CH_2=C-CO-$ ($CH_3$), $HS-CH_2-CH_2-CO-$,

$C_2H_5O-CO-CH_2-CH_2-$, $CH_3-CO-CH_2-CH_2-$, $CH_3O-CO-CH-CH_2-$ ($CH_3$),

**oder**

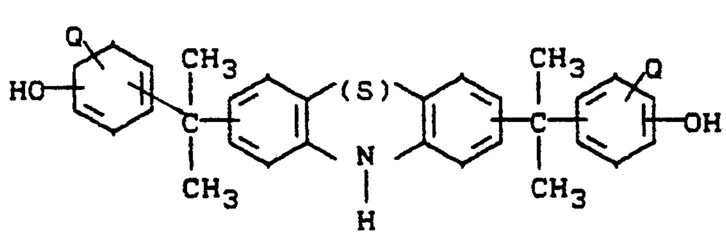

worin Q sein kann

$(CH_3)_3C-$, $CH_2=CH-CH_2-$, 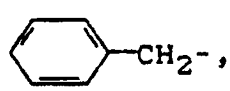, 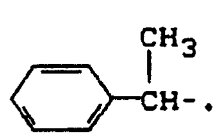.

Mit einem 2-bindigen Rest $R^3$ erhält man z.B.

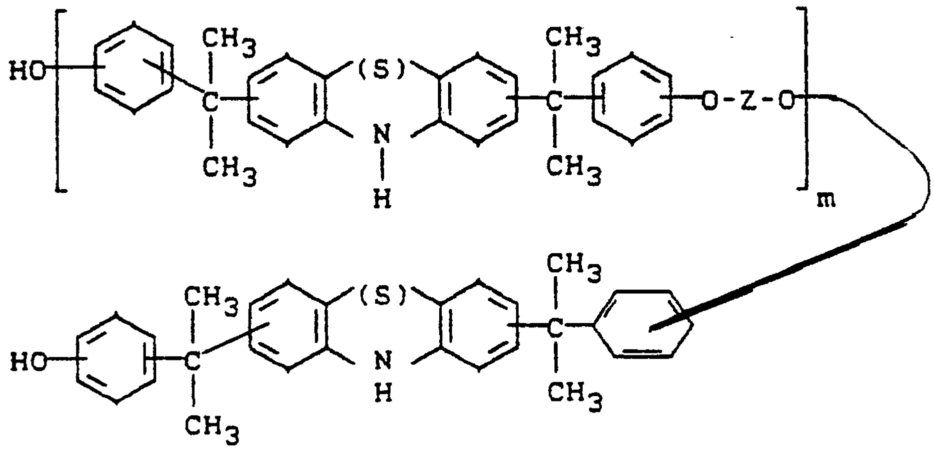

worin Z

$-CH_2-$, $-CH_2-CH_2-$, $-CH_2-CH{=}CH-CH_2-$, $-CH_2-\underset{OH}{CH}-CH_2-$,

$-CH_2-CH-CO-O-CH_2-CH_2-O-CO-CH_2-CH_2-$, $-CO-(-CH_2-)_4-CO-$,

$-CO-CH_2-O-CH_2-CO-$, $-CO-CH{=}CH-CO-$,

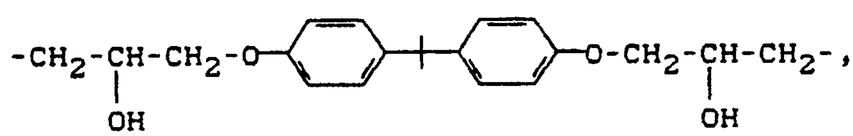

$-CO-CH_2-CH_2-S-CH_2-CH_2-CO-$ sein kann;

oder

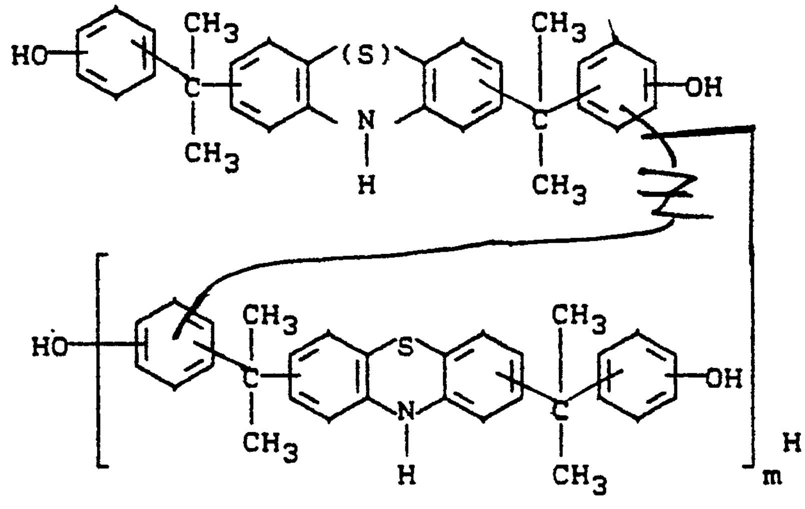

worin Z sein kann

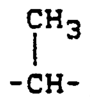, $-CH_2-$, 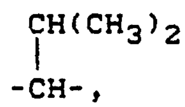, 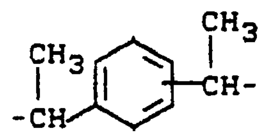,

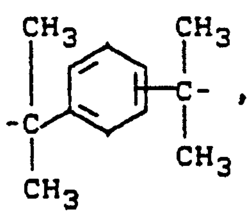,

und m eine ganze Zahl von 1 bis 15, vorzugsweise 1 bis 10 bedeutet.

Die über diese Reaktionen der primären Umsetzungsprodukte mit Verbindungen der Struktur (VIII) erhaltenen weiteren Umsetzungsprodukte sind gleichfalls Gegenstand der vorliegenden Erfindung.

Eine besondere Aufarbeitung der primären Umsetzungsprodukte oder der weiteren Reaktionsprodukte ist meist nicht erforderlich. Es genügt in der Regel den Katalysator zu entfernen und flüchtige Bestandteile abzudestillieren. Das Sumpfprodukt kann als solches eingesetzt werden oder in Sonderfällen durch Ausfällen oder Umkristallisieren aus geeigneten Lösungsmitteln gereinigt werden.

Sowohl die Umsetzungsprodukte der Diphenylamine (II) und Phenothiazine (III) als solche wie deren vorstehend beschriebene modifizierte Derivate werden als ausgezeichnete Stabilisatoren für Polyamide und Kautschuke sowie Polyamide und Kautschuke enthaltende Produkte verwendet.

Sie werden in stabilisierenden Mengen von 0,1 bis 10 %, bevorzugt 0,2 bis 6, besonders bevorzugt 0,3 bis 5 Gew.-%, bezogen auf zu stabilisierende Substrate, verwendet.

Mit den erfindungsgemäßen Stabilisatoren können synthetische Polyamide, wie sie durch Polykondensation von Diaminen mit Dicarbonsäuren, durch Polymerisation jvon Lactamen oder durch Polykondensation von Aminocarbonsäuren erhalten werden, stabilisiert werden. Bevorzugt werden aliphatische Polyamide, insbesondere solche aus Adipinsäure und Hexamethylendiamin oder aus Caprolactam bzw. solche Mischpolyamide, in denen die letztgenannten Komponenten die Hauptbestandteile darstellen, stabilisiert.

Mit Vorteil können auch solche Polyamide stabilisiert werden, die polymere oder kautschukähnliche Modifikatoren, wie sie im Stand der Technik in großer Zahl zur Verbesserung der Zähigkeit bekannt sind,

enthalten.

Die Einarbeitung kann sowohl vor oder während der Polymerisation als auch anschließend erfolgen, wobei die Stabilisatoren in Substanz oder als Lösung in einem inerten Lösungsmittel oder einem der Polyamid bildenden Ausgangsstoffe oder als Konzentrat in einem geeigneten Polymeren, bevorzugt in Polyamid zum Einsatz kommen. Die Einarbeitung erfolgt bevorzugt in die Polyamidschmelze unter Verwendung bekannter Mischvorrichtungen, wie Extrudern, Knetern, statischen Mischern und Rührern. Zusätzlich können den Polyamiden verschiedene üblicherweise verwendete Zuschläge unterschiedlicher Art wie Gleit- und Entformungsmittel, Nucleierungsmittel, Pigmente, Farbstoffe, verstärkende oder nicht verstärkende Füllstoffe wie Mineralfasern, Glas- und Asbestfasern, Mikrokugeln aus Glas, Nucleiermittel wie Talkum, Siliciumoxid oder Glimmer, Antistatika, Weichmachern und UV-Stabilisatoren beigegeben werden.

Die nach dem erfindungsgemäßen Verfahren stabilisierten Polyamide eignen sich hervorragend für die Herstellung von technischer Seide für Fischnetze, Treibriemen, Förderbändern, Reifencord oder Formkörpern, die einer thermischen Belastung bei freiem Zutritt von Luft oder Sauerstoff ausgesetzt sind. Sie sind auch in Form von Drähten für z.B. Spanndrähte im Obst- oder Weinbau oder für Weidezäune hervorragend geeignet.

Die neuen Alterungsschutzmittel können - sofern sie für eine radikalische Anbindung geeignete Gruppen enthalten - auf mehreren Wegen an Polymere angebunden werden, nämlich während der radikalischen Polymerisation der untengenannten Monomeren, vorzugsweise durch Pfropfung auf fertige Polymerisate, besonders bevorzugt aber während der Härtung und Vulkanisation der Polymeren.

Diese Reaktionen werden nach an sich bekannten Verfahren in Gegenwart der Verbindungen (I) in Masse, Emulsion, Lösung oder Dispersion, die Härtung oder Vulkanisation unter den üblichen Bedingungen und in Anwesenheit der bekannten Härtungs- und Vulkanisationssysteme durchgeführt.

Die erfindungsgemäßen Alterungsschutzmittel können auch nach bekannten Verfahren mit Vinylmonomeren zu Mischpolymerisaten mit Molgewichten von 1000 bis 30 000 mit einem erhöhten Gehalt von 5 bis 70 Gew.-% bevorzugt 10 bis 60 Gew.-% an (I) umgesetzt werden.

Ferner können die Alterungsschutzmittel (I) auch auf Polymere mit Molgewichten von 1000 bis 30 000 (Zahlenmittel), vorzugsweise von 2000 bis 20 000 aufgepfropft werden, so daß die Polymere einen Gehalt von 10 bis 60 Gew.-%, vorzugsweise von 10 bis 50 Gew.-% an gebundenem Alterungsschutzmittel enthalten. Derartige Verbindungen werden dann den hochmolekularen Polymeren zugesetzt und bilden ebenfalls migrationsfeste und schwer extrahierbare, wirksame polymere Alterungsschutzmittel. Sie werden in solchem Mengen den hochmolekularen Polymeren zugesetzt, daß die vorstehend genannten Konzentrationen an Alterungsschutzmittel im Gesamtpolymer erhalten werden. Dazu werden die das Alterungsschutzmittel gebunden enthaltenden Polymeren mit niederem Molgewicht in Mengen von 1 bis 25, bevorzugt 4 bis 20 Gew.-%, bezogen auf die hochmolekularen Polymeren, eingesetzt.

Geeignete Polymere mit niedrigen Molgewichten für derartige Pfropfreaktionen sind beispielsweise Polybutadiene, Polyisoprene, Mischpolymere von Butadien und/oder Isopren mit Styrol, Acrylnitril, Methylmethacrylat, Ethylacrylat, α-Methylstyrol, Piperylen, Hexadien-1,3, Ethylen, Propylen und Vinylacetat oder entsprechende Pfropfkautschuke.

Geeignete Vinylmonomere zur Herstellung der Mischpolymerisate sind die gleichen, wie sie vorstehend bereits genannt wurden.

Die Pfropfung der Alterungsschutzmittel auf die Polymeren erfolgt unter radikalischen Bedingungen, beispielsweise in Gegenwart bekannter Radikalstarter wie tert.-Butylperpivalat, Dicumylperoxid, Di-tert.-butylperoxid oder Azodiisobutyronitril, unverdünnt oder in inerten Lösungsmitteln wie Toluol, Xylol, Benzin, Chlorbenzol oder Dichlorbenzol, bei Temperaturen zwischen 50 und 200 °C, bevorzugt 70 bis 180 °C.

Die neuen Alterungsschutzmittel eignen sich gleichermaßen für eine breite Palette von Kautschuken und Kunststoffen, insbesondere aber für Kautschuke, z.B. für Polymere aus 1,3-Dienen wie Butadien, Isopren, Piperylen, 2-Chlorbutadien und/oder 2-Ethyl-butadien und deren Copolymere mit Vinylmonomeren, wie Styrol, p-Methylstyrol, α-Methylstyrol, Norbornen, Norbornadien, Acrylsäure, Acrylsäureester und -amide, Acrylnitril, Ethylen, Propylen und Vinylacetat, für Polyalkenamere, beispielsweise aus Cyclopenten oder 1,5-Cyclooctadien, und für Polymere aus 1-Olefinmischungen, beispielsweise aus Ethylen/Propylen oder Ethylen/Propylen/Dien mit isolierten Doppelbindungen. Solche Polymeren können durch radikalische, koordinative, metathetische oder ionische Polymerisation entstanden sein.

Beispiele derartiger Polymere sind BR, Naturkautschuk, SBR-, NBR-, EPDM- und CR-Kautschuk, Polypentenamer, ferner Polyethylen, Polypropylen oder Polystyrol mit geringen Gehalten an Doppelbindungen, schließlich auch ein- oder mehrphasige Polymermischungen, wie ABS oder Polystyrol, Polyethylen, Polypropylen, bevorzugt aber Doppelbindungen enthaltende Polymerisate.

Besonders wirkungsvoll sind die Alterungsschutzmittel bei Nitrilkautschuk.

Die Kautschuke können vulkanisiert sein.

Eine weitere Verbesserung der Bruchdehnung kann durch Zusatz von 5 bis 15 Gew.-%, bezogen auf Kautschukfeststoff, oligomerer Thioether, beispielsweise Etherthioether, wie Vulkanol 85® der Bayer AG, Leverkusen, erzielt werden.

Erfindungsgemäße Alterungsschutzmittel, die keine besonderen, für eine Polymerfixierung geeigneten Haftgruppen enthalten, werden auf übliche Weise mittels Kneter-, Walzen-, Schnecken- und Extrusionsgeräten in die Polymeren, die gegebenenfalls anschließend vulkanisiert werden, eingearbeitet.

Beispiel 1

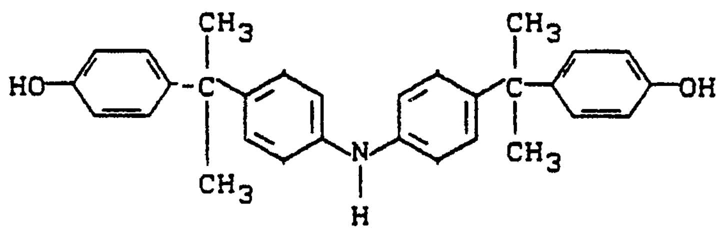

Ein Gemisch von 338 g (2,0 Mol) Diphenylamin, 268 g (1,0 Mol) dimerem p-Isopropenylphenol und 20 g Tonsil® Optimum wird 2 h auf 150° erhitzt, mit Xylol verdünnt und durch Druckfiltration von Katalysator befreit. Nach Eindampfen des Filtrats bis zu einer Sumpftemperatur von 190° bei 1 mbar erhält man 572 g eines hellbraunen spröden Harzes. Umkristallisieren aus Xylol/Ligroin (5:1) ergibt ein kristallines Produkt mit dem Schmelzpunkt 136-42°, das überwiegend der obigen Formel entspricht.

Beispiel 2

Ein Gemisch von 386 g (2,28 Mol) Diphenylamin, 1043 g (4,58 Mol) Bisphenol A und 40 g Tonsil® Optimum (Säureaktivierter Montmorillonit der Fa. Südchemie,München) wird unter Stickstoff aufgeschmolzen. Unter Rühren erhitzt man auf 150° und reduziert den Druck auf 10-20 mbar. Nun beginnt über eine ca. 50 cm lange, verspiegelte Vigreux-Kolonne langsam Phenol abzudestillieren. In dem Maße wie Phenol abdestilliert, erhöht man die Sumpftemperatur bis zum Schluß auf 195°. Nach 17-20 h ist die Reaktion beendet und 445 g Phenol sind übergegangen. Der Rückstand wird mit Toluol verdünnt, über eine Drucknutsche von Katalysator befreit, der Filterkuchen mit Toluol nachgewaschen und die vereinigten Filtrate bis zu einer Sumpftemperatur von 170° bei ca. 20 mbar eingedampft. Man erhält 963 g eines braunen spröden Harzes mit einem Gehalt an phenolischem OH von 7,7 Gew.-% (ber. 7,2 % bezogen auf Formel VII mit R und $R^1$ = H).

Beispiel 3

Ein Gemisch von 199 g (1,0 Mol) Phenothiazin, 456 g (2,0 Mol) Bisphenol A und 20 g Tonsil® Optimum wird wie in Beispiel 2 umgesetzt. Innerhalb von 14 h destillieren bis zu einer Sumpftemperatur von 205° und 15 mbar 181 g Phenol. Nach Aufarbeitung wie in Beispiel 2 erhält man 454 g eines braunen spröden Harzes mit einem Gehalt an phenolischem OH von 7,1 Gew.-%.

Beispiel 4

a)

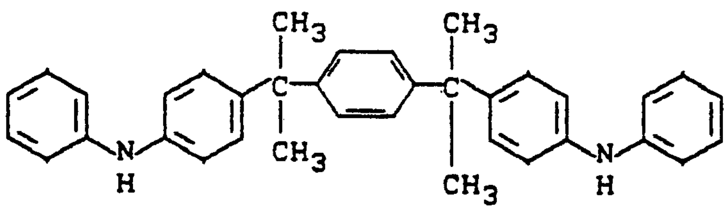

1352 g (8 Mol) Diphenylamin und 50 g säureaktivierte Tonerde werden unter $N_2$ und Rühren auf 100° gebracht und dazu 194 g (1 Mol) α,α'-Dihydroxy-p-diisopropylbenzol zugegeben. Man erhitzt bis 150°, wobei das Reaktionswasser abdestilliert. Man hält noch 1 h bei 150°, erhitzt für 30 Min. auf 170°, filtriert durch eine Drucknutsche und destilliert aus dem klaren Filtrat bei 1-2 mbar überschüssiges Diphenylamin ab. Der Sumpf wird in heißem Xylol aufgenommen. Beim Erkalten erhält man ein kristallines Produkt vom Schmelzpunkt 170-180°.

b) Ein Gemisch von 164 g (0,33 Mol) des Produktes aus dem vorhergehenden Ansatz 4a), 151 g (0,66 Mol) Bisphenol A und 10 g Tonsil® Optimum wird wie in Beispiel 2 in 12 h umgesetzt und eine Menge von 63 g Phenol abdestilliert. Nach Aufarbeitung bleiben 251 g eines braunen spröden Harzes.

Beispiel 5

a) Ein Gemisch von 338 g (2 Mol) Diphenylamin, 136 g (1 Mol) Limonen und 20 g Tonsil® Optimum wird 10 h auf 200° C gehalten, durch Filtration über eine Drucknutsche von Katalysator und durch Destillation bei ca. 1 mbar zu einer Sumpftemperatur von 200° C von überschüssigen Ausgangsprodukten befreit. Man erhält 331 g eines gelben weichen Harzes, dem laut Analyse die idealisierte Struktur

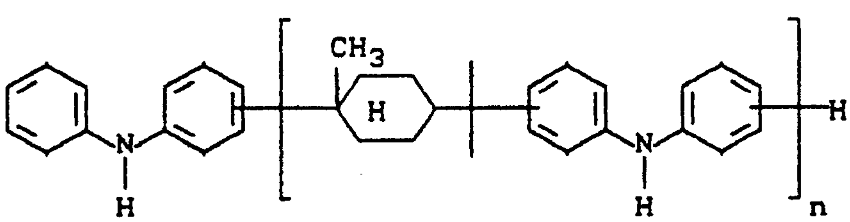

mit n im wesentlichen 1-5 zukommt.

b) Ein Gemisch von 196 g des Produktes aus dem Versuch 5a) 190 g Bisphenol A und 15 g Tonsil® Optimum wird wie in Beispiel 2 umgesetzt, wobei 73 g Phenol abdestilliert werden. Die Aufarbeitung ergibt 290 g gelbbraunen Harzes.

Beispiel 6

220 g (ca. 0,5 Mol) des Produktes aus Beispiel 2 werden in Ethanol gelöst, mit 15 g konzentrierter Salzsäure versetzt und unter Stickstoff und Rühren tropfenweise mit 27 g 37 gew.-%iger Formalinlösung versetzt.Nach 6 h bei 100° wird mit Ammoniaklösung schwach alkalisch gestellt. Das Reaktionsgemisch wird nach Verdünnen mit Toluol mit Wasser salzfrei gewaschen und bis 170° im Sumpf bei 20 mbar eingedampft. Man erhält 205 g eines spröden Harzes.

Beispiel 7

220 g (ca. 0,5 Mol) des Produktes aus Beispiel 2 werden in Isopropanol mit 20 g (0,5 Mol) NaOH gelöst und unter Rühren und Stickstoff zum Sieden erhitzt. In ca. 30 Minuten tropft man 47 g (0,5 Mol) Epichlorhyrin hinzu und kocht weiter 7 h unter Rückfluß, destilliert dann soviel Isopropanol ab, daß die Sumpftemperatur auf 135° steigt, die man für 3 h beibehält. Nach Druckfiltration zur Abtrennung des ausgefallenen Salzes (ca. 30 g) wird das Filtrat bis 170° im Sumpf bei 20 mbar eingedampft. Man erhält ein sprödes Harz (241 g).

Entsprechend einer idealisierten Struktur:

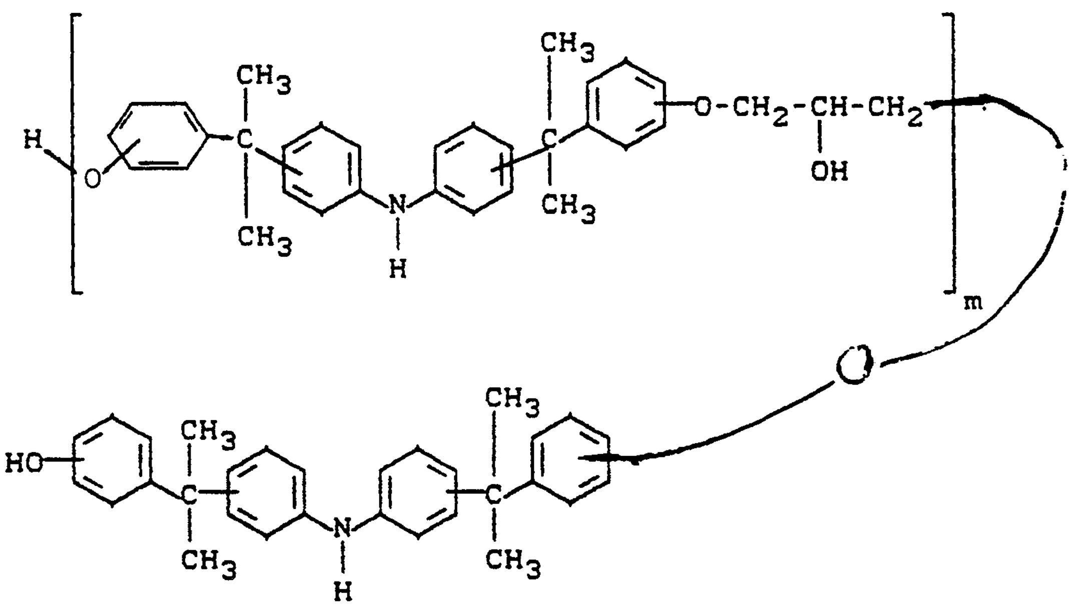

Beispiel 8

200 g (ca. 0,46 Mol) des Produktes aus Beispiel 2 werden mit 40 g (1,0 Mol) NaOH in Ethanol gelöst, unter Rühren und Stickstoff zum Sieden erhitzt und dann in ca. 30° mit 75 g (0,975 Mol) Allylchlorid tropfenweise versetzt. Nach weiteren 10 h bei 78° wird vom ausgefallenen Salz abgesaugt, das Salz (ca. 58 g) mit Ethanol digeriert und die vereinigten Filtrate nach Verdünnen mit Toluol mit Wasser völlig salzfrei gewaschen und bis zu einer Sumpftemperatur von 100° bei 10 mbar eingedampft. Man erhält 225 g eines hochviskosen hellbraunen Harzes, das nach Analyse zum O-Allylether des Produktes aus Beispiel 2 umgesetzt ist und (teilweise) der idealisierten Struktur entspricht.

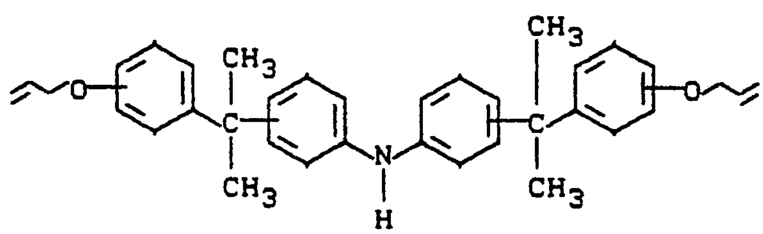

Vergleichsbeispiel 1

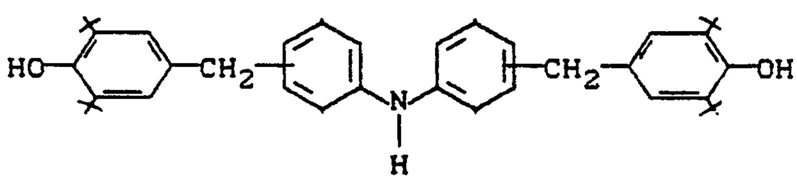

nach Beispiel 2 der US-PS 3 673 091.

Vergleichsbeispiel 2

Statt Diphenylamin wird Phenothiazin mit 4-Hydroxy-3,5-di-tert-butyl-benzylalkohol umgesetzt, (siehe auch Vergleichsbeispiel 1).

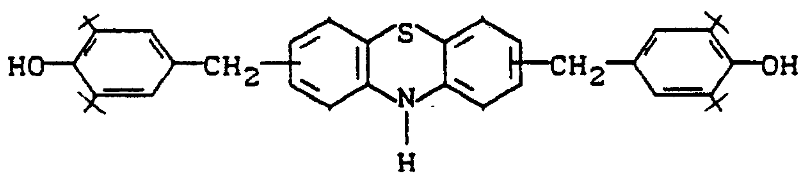

Prüfung der Stabilisatorwirkung in Polyamid-6

Zur Prüfung der Stabilisatorwirkung wurde die zu prüfende Substanz in einer Konzentration von 0,5 Gew.-% durch einen Extruderdurchgang homogen in 6-Polyamid ($\eta_{rel}$ = 4,0) eingearbeitet. Das so stabilisierte Material wurde zu Normkleinstäben verspritzt und einer Wärmealterung unter Luftzutritt bei 150 ± 0,5 °C unterworfen. Nach 1, 2, 4, 8 usw. Tagen wurden jeweils 8 Normkleinstäbe entnommen und nach zweistündiger Abkühlung im Exsiccator auf Schlagzähigkeit nach DIN 53 453 untersucht. Die Prüfung gilt als bestanden, wenn mindestens die Hälfte der Prüfkörper nicht gebrochen ist oder wenn die durchschnittliche Schlagzähigkeit der gebrochenen Stäbe oberhalb 30 kJ/m$^2$ liegt. Die Bewertung der Stabilisatorwirkung geschieht nach einer Stufeneinteilung entsprechend der folgenden Tabelle:

| Wärmealterungszeit bis zum Nicht-Bestehen der Schlagprüfung | 1 | 2 | 4 | 8 | 16 etc. Tagen |
|---|---|---|---|---|---|
| Wirksamkeitsstufe des Stabilisators | 0 | 1 | 2 | 3 | 4 |

Die Prüfungsergebnisse der erfindungsgemäßen Stabilisatoren finden sich in Tabelle I, die der Vergleichsprodukte in Tabelle II.

Tabelle I

| Stabilisator nach Beispiel | Wirksamkeitsstufe |
|---|---|
| 1 | 4-5 |
| 2 | 5 |
| 3 | 4 |
| **Vergleichsbeispiele** | |
| 1 | 1-2 |
| 2 | 1-2 |

Die erfindungsgemäßen Stabilisatoren übertreffen die Verbindungen nach US-PS 3 673 091 in jedem

Fall durch ihr weit höheres Wirkungsniveau.

Prüfung der Stabilisatorwirkung in NBR-Kautschuk

Ein NBR-Kautschuk aus 72 Gew.-% Butadien und 28 Gew.-% Acrylnitril wurde nach folgender Rezeptur in Anwesenheit von erfindungsgemäßen und bekannten Alterungschutzmittel vulkanisiert:

100,0 Gew.-Tle. NBR
1,5 Gew.-Tle. Mercaptosilan
30,0 Gew.-Tle. gefällte Kieselsäure
0,75 Gew.-Tle. Stearinsäure
3,0 Gew.-Tle. Zinkoxid
2,5 Gew.-Tle. eines Gemisches aus Fettsäure und Fettsäureestern
30,0 Gew.Tle. Kaolin calciniert
0,2 Gew.-Tle. Schwefelgranulat 80 %ig
2,5 Gew.-Tle. Tetramethylthiuramdisulfid
2,0 Gew.-Tle. Dibenzothiazyldisulfid
2,0 Gew..Tle. Mercaptobenzthiazol-Zinksalz
2,0 Gew.-Tle. Alterungsschutzmittel A-D.

A = Distyryldiphenylamin, handelsübliches, nicht gebundenes Alterungsschutzmittel (zum Vergleich)
B = Alterungsschutzmittel aus Beispiel 2
C = Alterungsschutzmittel aus Beispiel 8
D = Alterungsschutzmittel aus Beispiel 7

| | Vergleich | erfindungsgemäße Stabilisierung | | |
|---|---|---|---|---|
| | A | B | C | D |
| Mooney Scorch 120° C (min) | 14,0 | 14,0 | 14,0 | 14,0 |
| Vulkameter $t_{10}$ (min) | 2,8 | 2,8 | 2,7 | 2,7 |
| 170° C $t_{10}$ (min) | 4,2 | 4,2 | 4,1 | 4,1 |
| Vulkanisation 20' 170° C Heißluftalterung bei 135° C im Zellofen | | | | |
| Restbruchdehnung % nach 7 Tagen | 82 | 92 | 96 | 80 |
| Restbruchdehnung % nach 11 Tagen | 64 | 72 | 67 | 63 |
| Lagerung in Kraftstoff C 48 h 40°, Rücktrocknung 48 h 40° C im Vakuum, Heißluftalterung bei 135° C | | | | |
| Restbruchdehnung % nach 7 Tagen | 78 | 85 | 87 | 75 |
| Restbruchdehnung % nach 11 Tagen | 65 | 87 | 77 | 73 |

Die Tabelle zeigt deutlich:

a) Die erfindungsgemäßen Alterungsschutzmittel B und C übertreffen das bisher als hochwirksam angesehene, handelsübliche Produkt A bei 135° C-Heißluftalterung beträchtlich in der Wirkung. Das Alterungsschutzmittel D ist dem A vergleichbar.

b) Nach extraktiver Behandlung (Kraftstoff) und anschließender Alterung sind alle erfindungsgemäßen Alterungsschutzmittel dem Vergleich überlegen, wobei hervorzuheben ist, daß die Verbindung B sich besonders resistent gegen die extraktive Alterung vehrält, obwohl sie keine Haftgruppen für die Verankerung im Polymersubstrat besitzt.

## Patentansprüche

1. Umsetzungsprodukte aus Diphenylaminen der allgemeinen Struktur (II) und Phenothiazinen der allgemeinen Struktur (III)

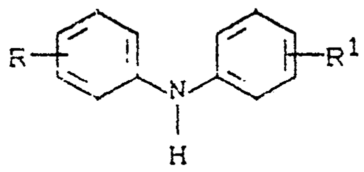

(II)

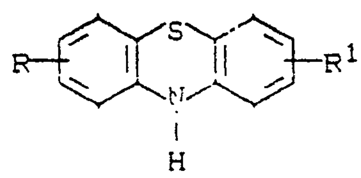

(III)

worin

R und $R^1$ gleich oder verschieden sind und in o-, m- und/oder p-Stellung zum N-Atom stehen und Wasserstoff, $C_1$- bis $C_{18}$-Alkyl-, $C_7$ bis $C_{10}$-Aralkyl-, OH-, Cl, einen Rest der Struktur (IIa)

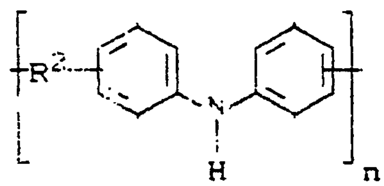

(IIa)

oder einen Rest der Struktur (IIIa)

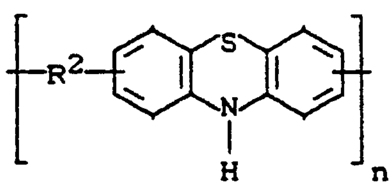

(IIIa)

bedeuten, in denen

$R^2$ ein Rest

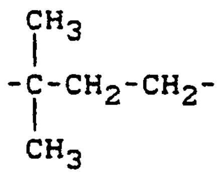 , 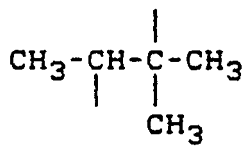 , 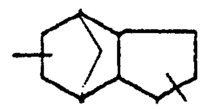 ,

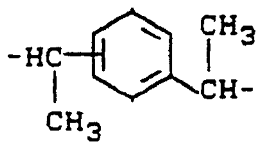 , 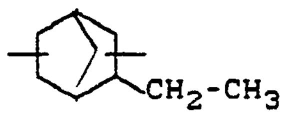 , 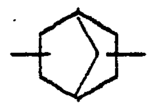 ,

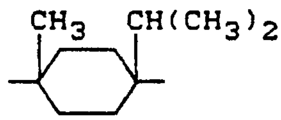 , 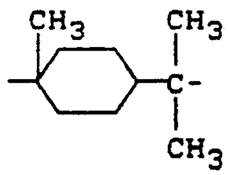 , 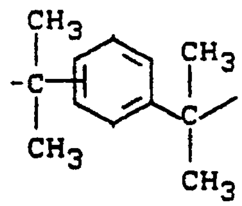

oder $-CH_2-$, ist,

wobei die Reste (IIa) und (IIIa) über $R^2$ mit den Resten (II) bzw. (III) verbunden sind und n eine ganze Zahl von 1 bis 5 bedeutet,
mit Isopropenylphenolen der allgemeinen Struktur (IV)

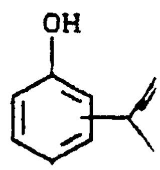 (IV)

worin die OH-Gruppe in o-, m- oder p-Stellung zur Isopropenylgruppe steht,

oder Bisphenolen der Struktur (V)

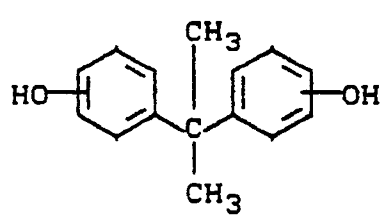 (V)

worin die OH-Gruppen in ortho-, meta- und/oder para-Stellung zur Isopropylidengruppe stehen, die durch Umsetzung von Aminen (II) und/oder (III) mit Phenolen (IV) und/oder (V) im Molverhältnis 0,1:1 bis 2 : 1 in Gegenwart von Säurekatalysatoren bei Temperaturen zwischen 100 und 300° gewonnen werden

und gegebenenfalls zusätzlich durch weitere Reaktionen mit Verbindungen der allgemeinen Struktur (VIII)

$X\{R^3-Y]$ (VIII)

worin

X und Y gleich oder verschieden sein können und H, OH, Halogen, eine Ethylen-, Propylen- oder eine Oxirangruppe bedeuten, wobei auch einer der beiden Substituenten oder der Ausdruck in der Klammer H sein kann, mit der Auflage, daß X dann von OH oder Halogen verschieden ist, ferner X -CH=O sein kann, wenn der Rest in der eckigen Klammer ein aliphatischer Rest mit 1 bis 4 Kohlenstoffatomen ist,

$R^3$ ein ein- oder zweibindiger aromatischer Rest oder ein ein- oder zweibindiger aliphatischer Rest mit 1 bis 12 C-Atomen, der eine oder mehrere OH-, Ether-, Thioether-, Carbonyl-, Estergruppen und/oder Doppelbindungen sowie aromatische Kerne mit 6 bis 12 C-Atomen enthalten kann, ist, wobei diese Reaktion sowohl an den OH-Gruppen wie an den aromatischen Kernen der primären Umsetzungsprodukte erfolgen können, modifiziert werden.

2. Umsetzungsprodukte von (II) und (III) nach Anspruch 1, in denen R und $R^1$ H, $CH_3$, $C_2H_5$, tert.-Butyl, Isooctyl, OH oder ein Rest der Struktur (IIa) oder (IIIa), worin $R^2$

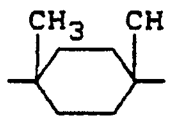 , 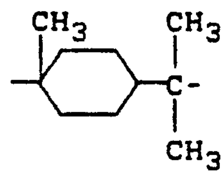 , 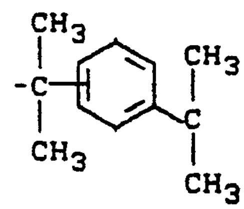 ,

oder $-CH_2-$, ist,

wobei die Reste (IIa) und (IIIa) über $R^2$ mit den Resten (II) bzw. (III) verbunden sind und n eine ganze Zahl von 1 bis 3 bedeutet,

mit Isopropenylphenolen oder Bisphenolen der Struktur (V)
und gegebenenfalls modifiziert durch weitere Reaktion mit X-($R^3$-Y) (VIII), worin X und Y = H, Halogen, OH, Ethylen, Propylen und Oxiran, X außerdem -HC=O, wenn der Rest in der Klammer H oder $CH_3$ ist, $R^3$ ein 1- oder 2-bindiger aromatischer Rest mit 6 C-Atomen oder ein 1 oder 2-bindiger aliphatischer Rest mit 1 bis 9 C-Atomen, der eine oder zwei Ether-, Estergruppen und/oder Doppelbindungen sowie aromatische Kerne mit 6 C-Atomen enthalten kann.

3. Umsetzungsprodukte nach Ansprüchen 1 und 2 von Diphenylamin und Phenothiazin mit Isopropenylphenolen (IV) oder Bisphenolen der Struktur (V), worin die OH-Gruppen in p-Stellung zur Isopropenylgruppe bzw. zur Isopropylidengruppen stehen und gegebenenfalls modifiziert durch weitere Reaktion mit (VIII), worin
X und Y H, Halogen, Oxiran
X außerdem -CH=O, wenn der Ausdruck in der Klammer H ist,
$R^3$ ein 1- oder 2-bindiger aliphatischer Rest mit 1 bis 6 C-Atomen ist.

4. Verwendung der Umsetzungsprodukte nach Ansprüchen 1 bis 3 in stabilisierenden Mengen von 0,1 bis 10 Gew.-%, vorzugsweise 0,2 bis 6 Gew.-%, insbesondere 0,3 bis 5 Gew.-%, zur Stabilisierung von Polyamiden oder Kautschuken und Kautschuk enthaltenden Materialien.

5. Polyamide enthaltend stabilisierende Mengen von 0,1 bis 10 Gew.-% an Umsetzungsprodukten nach Ansprüchen 1 bis 3.

6. Polyamid-6 und Polyamid-66, enthaltend stabilisierende Mengen von 0,1 bis 10 Gew.-%, vorzugsweise 0,2 bis 6 Gew.-% an Umsetzungsprodukten nach Ansprüchen 1 bis 3.

7. Kautschuke und Kautschuk enthaltende Materialien, enthaltend 0,1 bis 10 Gew.-%, vorzugsweise 0,2 bis 6 Gew.-% an Umsetzungsprodukten nach Ansprüchen 1 bis 3.

8. Kautschuke nach Anspruch 7, gekennzeichnet durch Verwendung von NBR-Kautschuk, enthaltend stabilisierende Mengen von 0,1 bis 10 Gew.-%, vorzugsweise 0,2 bis 6 Gew.-% an Umsetzungsprodukten nach Ansprüchen 1 bis 3.

## Claims

1. Reaction products of diphenyl amines corresponding to general formula (II) and phenothiazines corresponding to general formula (III)

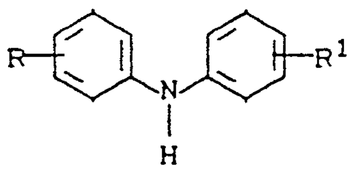

(II)

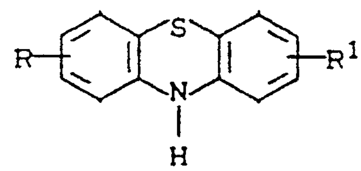

(III)

in which
R and $R^1$, which may be the same or different and are in the o-, m- and/or p-position to the N atom, represent hydrogen, $C_{1-18}$ alkyl, $C_{7-10}$ aralkyl, OH, Cl, a group having the formula

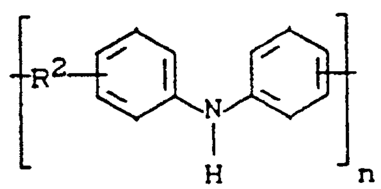

(IIa)

or a group having the formula

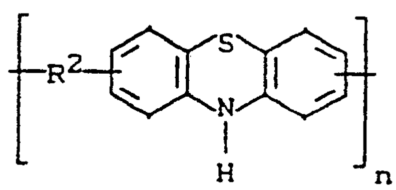

(IIIa)

in which
$R^2$ is one of the following groups

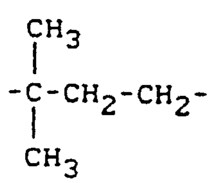 ,

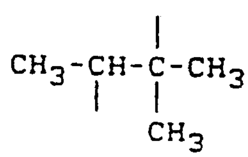 ,

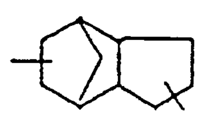 ,

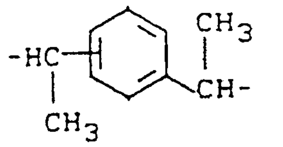 , 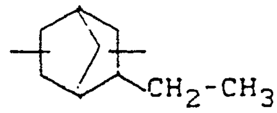 , 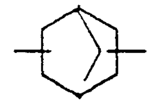 ,

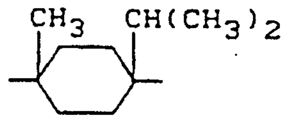 , 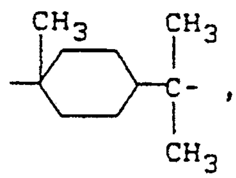 , 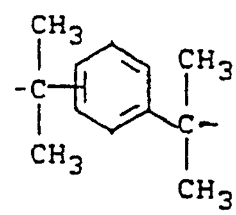

or $-CH_2-$,
the groups (IIa) and (IIIa) being attached by $R^2$ to the groups (II) and (III) and n being a number of 1 to 5, with isopropenyl phenols corresponding to the following general formula

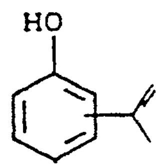 (IV)

in which the OH group is in the o-, m- or p-position to the isopropenyl group,
or bisphenols corresponding to formula (V)

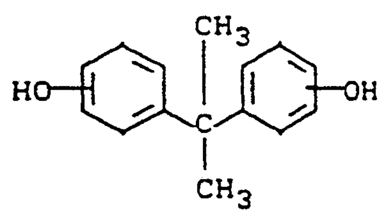 (V)

in which the OH groups are in the ortho-, meta- and/or para-position to the isopropylidene group,
which are obtained by reaction of amines (II) and/or (III) with phenols (IV) and/or (V) in a molar ratio of 0.1:1 to 2:1 in the presence of acid catalysts at temperatures of 100 to 300 °C
and, optionally, modified by further reactions with compounds corresponding to general formula (VIII)

$X[R^3-Y]$

in which
X and Y may be the same or different and represent H, OH, halogen, an ethylene, propylene or oxirane group; one of the two substituents or the expression in brackets may also be H, with the proviso that, in that case, X is not OH or halogen; X may also represent -CH=O when the expression in the square brackets is an aliphatic radical containing 1 to 4 carbon atoms,
$R^3$ is a single-bond or two-bond aromatic radical or a single-bond or two-bond aliphatic radical containing 1 to 12 carbon atoms which may contain one or more OH, ether, thioether, carbonyl, ester groups and/or double bonds and also aromatic nuclei containing 6 to 12 carbon atoms; these reactions may take place both at the OH groups and at the aromatic nuclei of the primary reaction products.

**2.** Reaction products of (II) and (III) as claimed in claim 1, in which R and $R^1$ represent H, $CH_3$, $C_2H_5$, tert. butyl, isooctyl, OH or a group corresponding to formula (IIa) or (IIIa), in which $R^2$ is

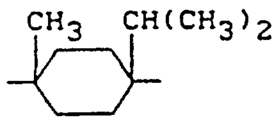 , 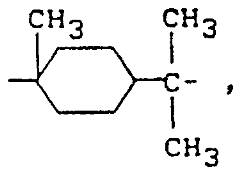 , 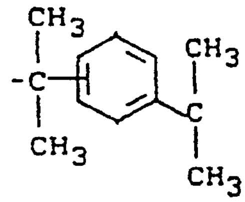 ,

or $-CH_2-$,
the groups (IIa) and (IIIa) being attached by $R^2$ to the groups (II) and (III) and m being an integer of 1 to 3, with isopropenyl phenols or bisphenols corresponding to formula (V) and optionally modified by further reaction with X-($R^3$-Y) (VIII), in which X and Y = H, halogen, OH, ethylene, propylene and oxirane and, in addition, X may represent -HC=O where the expression in brackets is H or $CH_3$,
$R^3$ is a single-bond or two-bond aromatic radical containing 6 carbon atoms or a single-bond or two-bond aliphatic radical containing 1 to 9 carbon atoms which may contain one or two ether groups, ester groups and/or double bonds and also aromatic nuclei containing 6 carbon atoms.

3. Reaction products as claimed in claims 1 and 2 of diphenyl amine and phenothiazine with isopropenyl phenols (IV) or bisphenols (V), in which the OH groups are in the p-position to the isopropenyl group or to the isopropylidene group, and optionally modified by further reaction with (VIII), in which
X and Y are H, halogen, oxirane, and, in addition,
X may represent -CH=O where the expression in brackets is H,
$R^3$ is a single-bond or two-bond aliphatic radical containing 1 to 6 carbon atoms.

4. The use of the reaction products claimed in claims 1 to 3 in stabilizing quantities of 0.1 to 10% by weight, preferably 0.2 to 6% by weight and, more preferably, 0.3 to 5% by weight for stabilizing polyamides or rubbers and rubber-containing materials.

5. Polyamides containing stabilizing quantities of 0.1 to 10% by weight of the reaction products claimed in claims 1 to 3.

6. Polyamide-6 and polyamide-66 containing stabilizing quantities of 0.1 to 10% by weight and preferably 0.2 to 6% by weight of the reaction products claimed in claims 1 to 3.

7. Rubbers and rubber-containing materials containing 0.1 to 10% by weight and preferably 0.2 to 6% by weight of the reaction products claimed in claims 1 to 3.

8. Rubbers as claimed in claim 7, characterized by the use of NBR rubber containing stabilizing quantities of 0.1 to 10% by weight and preferably 0.2 to 6% by weight of the reaction products claimed in claims 1 to 3.

**Revendications**

1. Produits de réaction de diphénylamines de structure générale (II) et de phénothiazines de structure générale (III)

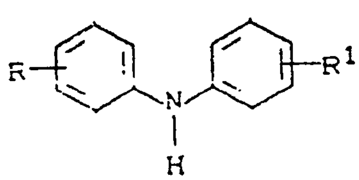

(II)

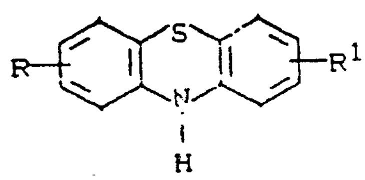

(III)

où

R et $R^1$ sont identiques ou différents et sont en position ortho, méta et/ou para par rapport à l'atome d'azote et représentent l'hydrogène, des groupes alkyle en $C_1$ à $C_{18}$, aralkyle en $C_7$ à $C_{10}$, OH-, Cl, un reste de structure (IIa)

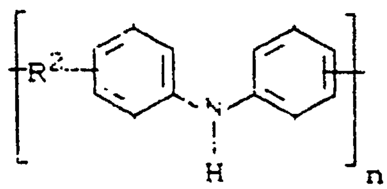 (IIa)

ou un reste de structure (IIIa)

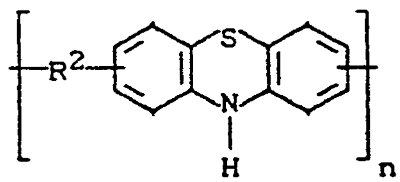 (IIIa)

où

$R^2$ est un reste

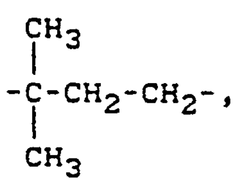, 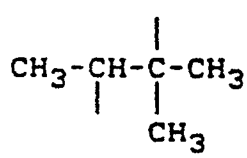, 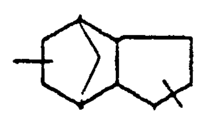,

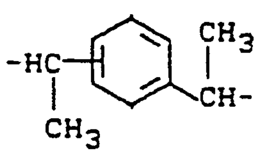, 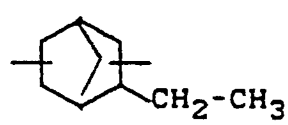, 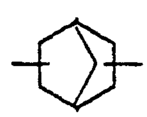,

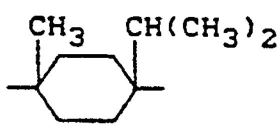, 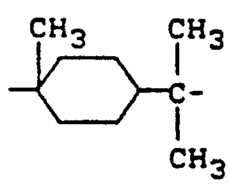, 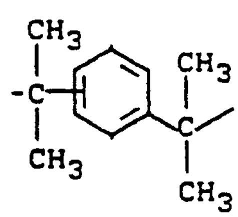

ou $-CH_2-$,

les restes (IIa) et (IIIa) étant liés par l'intermédiaire de $R^2$ aux restes (II) ou (III) et n représentant un nombre entier de 1 à 5,

avec des isopropénylphénols de structure générale (IV)

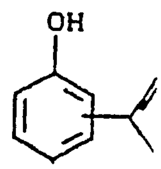

(IV)

dans laquelle le groupe OH est en position ortho, méta ou para par rapport au groupe isopropényle, ou des bisphénols de structure (V)

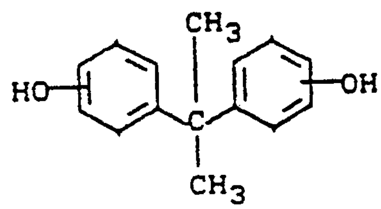

(V)

dans laquelle les groupes OH sont en position ortho, méta et/ou para par rapport au groupe isopropylidène, qui sont obtenus par réaction d'amines (II) et/ou (III) avec des phénols (IV) et/ou (V) dans le rapport molaire de 0,1:1 à 2:1 en présence de catalyseurs acides à des températures comprises entre 100 et 300°, et sont modifiés en outre le cas échéant par d'autres réactions avec des composés de structure générale (VIII)

$X[R^3-Y]$ (VIII)

dans laquelle

X et Y peuvent être identiques ou différents et représentent H, OH, un halogène, un groupe éthylène, propylène ou un groupe oxiranne, l'un des deux substituants ou l'expression placée entre parenthèses pouvant aussi représenter H, sous réserve que X soit différent de OH ou d'un halogène, et que X puisse en outre représenter -CH=O lorsque le reste placé entre crochets est un reste aliphatique ayant 1 à 4 atomes de carbone,

$R^3$ est un reste aromatique monovalent ou divalent ou un reste aliphatique monovalent ou divalent ayant 1 à 12 atomes de carbone, qui peut contenir un ou plusieurs groupes OH, éther, thioéther, carbonyle, ester et/ou doubles liaisons ainsi que noyaux aromatiques ayant 6 à 12 atomes de carbone, cette réaction pouvant s'effectuer tant sur les groupes OH que sur les noyaux aromatiques des produits de réaction primaires.

2. Produits de réaction de (II) et (III) suivant la revendication 1, dans lesquels R et $R^1$ représentent H, $CH_3$, $C_2N_5$, un groupe tertio-butyle, iso-octyle, OH ou un reste de structure (IIa) ou (IIIa), où $R^2$ représente

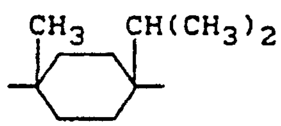 , 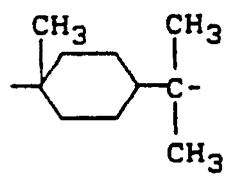 , 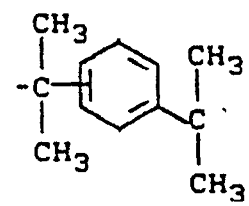 ,

ou $-CH_2-$

les restes (IIa) et (IIIa) étant liés par l'intermédiaire de $R^2$ aux restes (II) ou (III) et n désignant un nombre entier de 1 à 3,

avec des isopropénylphénols ou bisphénols de structure (V)

et le cas échéant modifiés par réaction ultérieure avec $X-(R^3-Y)$ (VIII), où X et Y représentent H, un halogène, un groupe OH, éthylène, propylène et oxiranne, X représente en outre -HC=O lorsque le reste entre parenthèses représente H ou $CH_3$,

$R^3$ est un reste aromatique monovalent ou divalent ayant 6 atomes de carbone ou un reste aliphatique monovalent ou divalent ayant 1 à 9 atomes de carbone, qui peut contenir un ou deux groupes éther, ester et/ou doubles liaisons ainsi que noyaux aromatiques ayant 6 atomes de carbone.

**3.** Produits de réaction suivant les revendications 1 et 2 de la diphénylamine et de la phénothiazine avec des isopropénylphénols (IV) ou des bisphénols de structure (V), les groupes OH étant en position para par rapport aux groupes isopropényle ou par rapport aux groupes isopropylidène et modifiés le cas échéant par réaction ultérieure avec (VIII), où

X et Y représentent H, un halogène, un groupe oxiranne

X représente en outre un groupe -CH=O lorsque l'expression entre parenthèses représente H,

$R^3$ est un reste aliphatique monovalent ou divalent ayant 1 à 6 atomes de carbone.

**4.** Utilisation des produits de réaction selon les revendications 1 à 3 en quantités stabilisantes de 0,1 à 10 % en poids, de préférence de 0,2 à 6 % en poids, notamment de 0,3 à 5 % en poids pour la stabilisation de polyamides ou de caoutchoucs et de matériaux contenant un caoutchouc.

**5.** Polyamides contenant des quantités stabilisantes de 0,1 à 10 % en poids de produits de réaction selon les revendications 1 à 3.

**6.** Polyamides-6 et polyamides-66 contenant des quantités stabilisantes de 0,1 à 10 % en poids, de préférence de 0,2 à 6 % en poids, de produits de réaction selon les revendications 1 à 3.

**7.** Caoutchoucs et matériaux contenant du caoutchouc, renfermant 0,1 à 10 % en poids, de préférence 0,2 à 6 % en poids de produits de réaction selon les revendications 1 à 3.

**8.** Caoutchoucs suivant la revendication 7, caractérisés par l'utilisation de caoutchoucs NBR, contenant des quantités stabilisantes de 0,1 à 10 % en poids, de préférence de 0,2 à 6 % en poids, de produits de réaction selon les revendications 1 à 3.